# EUROPEAN PATENT SPECIFICATION

(11) **EP 2 533 821 B1**
(45) Date of publication and mention of the grant of the patent: **13.07.2016**
(21) Application number: 11703455.3
(22) Date of filing: 11.02.2011
(51) Int. Cl.: A61L 27/28, A61L 27/30, A61L 27/54, A61L 27/56, A61L 31/08, A61L 31/14, A61L 31/16, A61F 2/06, C08L 27/18, A61L 27/16, A61L 27/50, A61L 31/04

(54) **MEDICAL DEVICE MADE OF EPTFE PARTIALLY COATED WITH AN ANTIMICROBIAL MATERIAL**
MEDIZINISCHE VORRICHTUNG AUS TEILWEISE MIT ANTIMIKROBIELLEM MATERIAL BESCHICHTETEM EPTFE
DISPOSITIF MÉDICAL EN EPTFE, PARTIELLEMENT ENDUIT D'UN MATÉRIAU ANTIMICROBIEN

(30) Priority: 12.02.2010 US 303990 P
(43) Date of publication of application: 19.12.2012
(73) Proprietor: Aesculap AG, 78532 Tuttlingen/Donau (DE); Spire Biomedical, Bedford, MA 01730 (US)
(72) Inventor: GOLDMANN, Helmut, 34119 Kassel (DE); LANGANKE, Dennis, 72074 Tübingen (DE); OLIVER, Richard W., Westford MA 01886 (US)
(74) Representative: Patentanwälte Ruff, Wilhelm, Beier, Dauster & Partner mbB
(86) International application number: PCT/EP2011/052043
(87) International publication number: WO 2011/098565

(56) References cited:
- EP-A1- 1 186 309
- WO-A1-2004/000375
- US-A- 4 955 899
- US-A- 5 782 789

## Description

The present invention relates to a medical device that is based on a porous article of expanded polytetrafluoroethylene (ePTFE) and a method for manufacturing the medical device.
Microbial colonisation and subsequent infections of vascular grafts are a well recognized complication in the field of vascular surgery. In particular, such infections are often associated with a high rate of amputation and mortality.
It is well known to use extruded tube structures of polytetrafluoroethylene (PTFE) as an implantable material as it exhibits superior biocompatibility. Particularly, PTFE tube structures may be used as vascular grafts in the replacement or repair of a blood vessel as PTFE exhibits low thrombogenicity. In vascular applications, the grafts are generally manufactured from expanded polytetrafluoroethylene (ePTFE) tube structures. These tube structures have a microporous microstructure which is defined by interspaced nodes interconnected by elongated fibrils. On the one hand, this microstructure may be selective adjusted by appropriate expanding and stretching conditions during the manufacturing of ePTFE tubes to lower the risk of thrombosis and to promote tissue ingrowth from the outer periphery of the graft. On the other hand, the microstructure of ePTFE is a labile structure which makes it difficult and complicated to equip ePTFE grafts with antimicrobial properties without destroying the node and fibril microstructure of ePTFE.

An ePTFE vascular prosthesis, which was subjected to a surface treatment with an antithrombogenic substance, is known from EP 1 186 309 A1.

US 4,955,899 discloses a longitudinally compliant ePTFE vascular graft comprising a coating of a biocompatible elastomer covering the outer surface of the vascular graft.

Accordingly, it is an object of the present invention to provide a medical device made of ePTFE having antimicrobial properties, in particular long-lasting antimicrobial properties, and additionally having an intact microstructure of nodes that are interconnected by fibrils. Furthermore, the medical device, in particular its microstructure, shall resist mechanical forces that typically occur in a human or animal body without being damaged or destroyed. It is a further object of the present invention to provide a medical device comprising a reduced amount of an antimicrobial material and nonetheless having a sufficient antimicrobial effect.

This problem is solved by a medical device having the features of independent claim 1. Preferred embodiments of the device are the object of dependent claims 2 to 11. A further aspect of the invention relates to a method for the manufacture of the medical device according to independent claim 12. Preferred embodiments of the method are the object of dependent claims 13 and 14. The wording of all of the claims is incorporated in the present description by reference.

The medical device according to the present invention comprises a porous article of expanded polytetrafluoroethylene (ePTFE) having a microstructure of interspaced nodes interconnected by fibrils and having a coating including an antimicrobial material at the surface of the porous article, wherein the microstructure is only partially coated with the antimicrobial material at the surface of the porous article.

The term "polytetrafluoroethylene" as used herein may also comprise a copolymer of tetrafluoroethylene.

The term "copolymer" as used herein preferably means a polymer that comprises at least two different monomer units.

Accordingly, the term "copolymer of tetrafluoroethylene" as used herein encompasses a polymer that is composed of at least one further monomer unit along tetrafluoroethylene. Such a further monomer unit may be selected from the group consisting of chlorotrifluoroethylene, perfluoroalkoxy- tetrafluoroethylene, hexafluoropropylene, tetrafluoropropylene and combinations thereof.

Only nodes or node segments at the surface of the porous article are coated, in particular completely coated, with the antimicrobial material. The fibrils at the surface of the porous article are at most partially coated with the antimicrobial material. More preferably, the fibrils of the microstructure, in particular fibrils at the surface of the porous article, are free of a coating including an antimicrobial material. Coating or predominantly coating of the fibrils would lead to an undesired stiffening and to undesired brittleness of the fibrils being more susceptible to breakage under mechanical forces that typically appear when the medical device is, for example, bent, stretched or compressed.

According to an especially preferred embodiment, the nodes being present at the surface of the porous article are coated with the antimicrobial material and the fibrils of the microstructure, in particular at the surface of the porous article, are free of a coating including an antimicrobial material. Such a device advantageously resists more easily mechanical forces, in particular bending forces, expanding forces and compressing forces, that may typically occur in a patient's body without damaging or destroying the microstructure. Furthermore, it may be within the scope of the present invention to provide a medical device having alternating antimicrobial properties at its surface (preferably nodes coated and interconnecting fibrils uncoated), nonetheless the surface may largely exercise an antimicrobial effect, in particular a long-lasting antimicrobial effect, that is sufficient to reduce the risk of post-surgical infections.

In a further preferred embodiment, the coating including an antimicrobial material leaves internodal spaces open of the microstructure, in particular at the surface of the porous article. Thus, natural tissue ingrowth is possible into the article from its outer periphery leading to a secure anchoring of the article within the body of a human or animal patient and facilitating a rebuilding of the original anatomical circumstances around the implantation region. The term "internodal space" as used herein refers to the space between the node surfaces that is spanned by the fibrils.

Generally, the nodes of the microstructure may have an elongate, in particular an ellipsoidal, shape. More preferably, the nodes have a relatively uniform shape and in particular uniform size. The nodular size, in particular the nodular length, may range from 1 to 800 µm, in particular from 5 to 500 µm, and preferred from 10 to 300 µm. The interconnecting fibrils may have a bent, wavy or straight appearance. More preferably, the fibrils of the microstructure that interconnect nodes may have a straight and in particular parallel appearance. Further, the fibrils may have a length from 1 to 150 µm, in particular from 20 to 80 µm. Furthermore, the fibrils may have a diameter that ranges from 0.1 to 10 µm, in particular from 0.5 to 5 µm. Typically, the microstructure may have internodal spaces from 1 to 150 µm, in particular from 10 to 100 µm, and preferred from 20 to 80 µm.

Further, the coating including an antimicrobial material may have a proportion from 0.01 to 5.0 % by weight, in particular from 0.1 to 3.0 % by weight, and preferred from 0.3 to 1.5 % by weight, related to the total weight of the porous article. Furthermore, the coating including an antimicrobial material, taken at the thickness, in particular wall thickness, of the porous article may have a proportion from 0.001 to 0.2 %, in particular from 0.004 to 0.125 %, and preferred from 0.01 to 0.04 %. In a further embodiment, the coating including an antimicrobial material is arranged in a depth from 1 to 150 µm, in particular from 20 to 100 µm, preferably from 40 to 80 µm, taken at the surface of the porous article.
In a preferred embodiment, the coating including an antimicrobial material is of such thickness that in vivo, i.e. after implantation, it has a dwell time on the article's surface of more than 1 month, particularly more than 6 months, and releases the antimicrobial material during this time. Preferably, the coating including an antimicrobial material has a layer thickness from 10 to 400 nm, in particular from 40 to 250 nm, and preferred from 80 to 160 nm.

In a further embodiment, the microstructure includes nodes that are arranged in differing internodal spaces. Preferably, the internodal spaces are arranged in a gradient. More preferably, the internodal spaces of the microstructure increase, in particular gradually increase, from one surface of the porous article to an opposing surface. It is especially preferred, that the internodal spaces increase, in particular gradually increase, from an inside surface to an outside surface of the porous article. It is further preferred that internodal spaces are sufficiently large enough at one surface, in particular at an outside surface, of the porous article, to allow natural tissue ingrowth into the article from its outer periphery. In contrast, it is especially preferred that internodal spaces are sufficiently small enough at a surface opposing the afore-mentioned one surface, preferably at an inside surface of the article, that substantially prevents natural tissue ingrowth or platelet activation and platelet adhesion but may facilitate the passage of low molecular compounds, for example nutrients, biological agents, medicinal agents et cetera.

The coating may further substantially consist of the antimicrobial material. However, it is within the scope of the present invention that the coating may include minor amounts of additional components, like pharmaceutical agents, antimicrobial agents, disinfectants, biological agents, radiopaque materials et cetera.

According to a further embodiment, the antimicrobial material is selected from the group consisting of an antimicrobial metal, antimicrobial alloy, antimicrobial compound, antimicrobial salt, in particular oxide, and combinations thereof. Antimicrobial metals, antimicrobial alloys and/or anitmicrobial metal compounds, in particular antimicrobial metal salts, are especially preferred. The antimicrobial material may be selected from the group consisting of zirconium, copper, zinc, silver, gold, palladium, platinum, iridium, aluminium, nickel, tungsten, molybdenum, tantalum, titanium, iodine, alloys thereof, compounds thereof, salts thereof, for instance oxides thereof, and combinations thereof. Silver is especially preferred. As used herein, the term "antimicrobial material" refers to a material which inhibits the growth of micro-organisms, such as pathogenic bacteria, protests and/or fungi which may cause serious infections within a patient.

In a further embodiment, the porous article comprise and adhesion-promoting agent. Typically, the adhesion-promoting agent is adjacent to the surface of the porous article. Preferably, the adhesion-promoting agent is arranged in the form of a layer. Further, the adhesion-promoting agent may be a metal, in particular titanium. The porous article may further comprise a species, in particular a layer thereof, which forms a barrier to degradation or diffusion of an adhesion-promoting agent and, for instance, impede galvanic interaction between the antimicrobial material and an adhesion-promoting agent. The species may be a metal, in particular palladium. Preferably, the barrier species is adjacent to the surface of the porous article and is in particular interposed between an adhesion-promoting agent and the antimicrobial material.

In a further embodiment, the porous article may comprise a leak-proofing coating or impregnation. Such a coating and impregnation, respectively is typically designed on the surface of the article. Generally, such a coating and impregnation, respectively seals internodal spaces of the microstructure at the surface of the porous article. Suitable materials for the coating and impregnation, respectively may be biological polymers and/or synthetic polymers, in particular copolymers. Synthetic polymers, in particular copolymers, based on hydroxyl acids are especially preferred. Suitable materials may be selected from the group consisting of collagen, gelatine, albumin, carboxymethylcellulose, polyvinylalcohol, polylactide, polyglycolide, poly-ε-caprolactone, poly-trimethylencarbonate, poly-para-dioxanone, copolymers thereof and combinations thereof. By suitable choice of the polymers, the desired duration of absorption may be set. This is preferably within four months and in particular within 40 days. Such a time is normally expedient, since depending on the type of the medical device, the impregnation action is no longer necessary during this time because of the incurring connective tissue.

In a especially preferred embodiment, the coating including an antimicrobial material is deposited onto the surface of the porous article by means of a surface treatment, in particular by means of a dry coating process. More preferably, the coating including an antimicrobial material is vapour deposited, in particular ion-beam-assisted deposited, onto the surface of the porous article.

According to an especially preferred embodiment, the porous article is a tubular article, typically a hollow tubular article. In other words, the porous article is preferably designed as a tubing, typically having a lumen encased by the tubing wall. Preferably, the coating including an antimicrobial material is present at the outside surface of the tubular article. In particular, only nodes or node segments at the outside surface of the tubular article are coated, in particular completely coated, with the antimicrobial material. The fibrils of the microstructure, in particular fibrils at the outside surface of the tubular article, are free of a coating including an antimicrobial material. Internodal spaces, in particular at the outside surface of the tubular article, are preferably free of a coating including an antimicrobial material. Furthermore, the tubular article may have different pore sizes, particularly internodal spaces, at its inside and outside surface. Preferably, the inside surface of the tubular article is basically arranged smooth. In other words, the microstructure at the inside surface of the tubular article preferably offers no or only a negligible resistance to flow of blood, and consequently, platelet activation and platelet adhesion may be reduced. Furthermore, it is preferred that the inside surface may have a pore structure, in particular internodal spaces, facilitating the passage of small molecular compounds, in particular of nutrients, biological agents and/or medical agents, through the wall of the tubular article into the lumen thereof. Thus a nutrition supply to a neointima is possible that preferably lines the insides surface of the tubular article. Thus, it is possible to greatly reduce calcification of neointima that may result from nutritional deficiency. Further, biological and/or medical agents may enter the lumen of the tubular article and, for instance, may help to prevent the manifestation of a thrombosis. Preferably, the microstructure at the outside surface of the tubular article promotes tissue ingrowth, in particular ingrowth of fibroblasts, from the outer periphery of the article. This contributes to a secure anchoring of the article with surrounding body tissue. Furthermore, the aforementioned nutrient supply is essentially based on capillaries which densely develop on fully grown fibroblasts. Preferably, pore sizes, in particular internodal spaces, of the microstructure increase, particularly gradually increase, from the inside surface to the outside surface of the tubular article.

In a further embodiment, the porous article is designed as a tubular article having an internal diameter from 2 to 50 mm.

In a further embodiment, the porous article, in particular a tubular porous article, has a thickness, in particular a wall thickness, from 0.1 to 1.0 mm, in particular from 0.25 to 0.75 mm, preferably from 0.4 to 0.6 mm.

In a further embodiment, the porous article may be designed as a two-dimensionally shaped (planar) article, in particular as a mesh, sling, patch and the like. Accordingly, it is in principle within the scope of the present invention that the medical device is designed as a hernia mesh, prolaps mesh or aconuresis sling.

In an especially preferred embodiment, the porous article is a tubular prosthesis. Preferably, the porous article is an arterial prosthesis or a vein prosthesis. More preferably, the porous article is designed as an arterial prosthesis.

Further, the medical device may be on hand in a sterile form and in particular tailored form.

The present invention further encompasses a method for the manufacture of a medical device according to the present invention comprising the steps of
a) compressing a porous article, preferably a tubular porous article, of expanded polytetrafluoroethylene (ePTFE) having a microstructure of interspaced nodes interconnected by fibrils,
b) coating the compressed porous article, preferably tubular porous article, with an antimicrobial material,
c) expanding the coated porous article, preferably tubular porous article.

Due to the compressing step a) the microstructure of the article is also compressed, i.e. the nodes are transferred into a compact state minimizing and in particular removing internodal spaces. Thus, basically only the nodes at the surface of the porous article are coated with the antimicrobial material. The fibrils of the microstructure are basically not coated with the antimicrobial material. As already mentioned, coated fibrils have an increased stiffness and brittleness thus being more susceptible to breakage under mechanical forces leading at least to a partially destruction of the node and fibril microstructure. The method of the present invention facilitates a partially coating of the porous article maintaining the original node and fibril microstructure of the porous article. This effects unimpaired flexibility of the porous article and the medical device, respectively and a better resistance to mechanical forces typically occurring in a patient's body.

In a preferred embodiment, the porous article is compressed on a mandrel, in particular on a rotatable mandrel. Due to a rotatable mandrel, an equally coating of the porous article with the antimicrobial material is possible. Preferably, the mandrel is designed as a rod. Furthermore, the mandrel may be manufactured from metal, steel or polymer, in particular polyethylene or polyvinylalcohol. In particular, the mandrel may have a diameter, in particular a radial diameter, from 2 to 50 mm.

In a further embodiment, the compressed porous article is coated with the antimicrobial material by means of a dry coating process, in particular by means of a physical vapour deposition process (PVD process), preferably an ion-beam-assisted deposition process (IBAD process).

In general, ion-beam processes are low-temperature, high-technology processes with excellent quality control to achieve good adherence, ductility, reproducibility, reliability and thickness of deposition control at a high throughput and with no chemical residues, thus being both environmentally and occupationally a safe dependable technique. Typically, an ion-beam-assisted coating apparatus comprises a vacuum chamber system formed of a low-vacuum antechamber and a high-vacuum processing chamber, air-tightly separated from each other by a gate movable between an opened position and a closed position. An ion source, which can be a bucket-type ion source is mounted within the high-vacuum processing chamber, normally in a position diametrically opposed to the low-vacuum antechamber. The ion source is typically fed by one or more gases, such as argon, oxygen, neon, and/or helium, from a suitable gas supply source via a mass flow controller, regulating the rate of gas feed.

Further, an evaporator is also mounted in the high-vacuum processing chamber, normally in operative association with the ion source. The evaporator is designed to vaporize particular evaporants, more specifically metallic evaporants, so as to dry-coat a specific substrate, in particular a medical device, therewith, being assisted in the dry-coating by an ion-beam emanating from the ion source. Suitable evaporants include zirconium, copper, zinc, silver, gold, palladium, platinum, iridium, aluminium, nickel, tungsten, molybdenum, tantalum, titanium, and their respective alloys, salts and compounds. Normally, a vapour shutter, designed to be rotated in and out of place of the evaporator, shields the substrate from the evaporants when in place. The substrate to be dry-coated is normally introduced into the vacuum chamber system with the aid of a suitable substrate holder. Preferably, the substrate holder is mounted for both rotational and translatory motion on a shaft and is introduced in the antechamber through a hinge-like mounted end-blade.

With respect to the above-made comments, the IBAD process that is preferably scheduled by the present invention is typically performed in a suitable vacuum chamber system including a processing chamber. The required vacuum environment is normally created by means of a vacuum. Preferably, the ion-beam-assisted deposition is performed under a vacuum pressure of at least 10⁻⁴ torr, in particular of at least 10⁻⁵ torr. Further, the ion-beam-assisted deposition may be performed at a temperature < 150 °C. Moreover, the porous article may be exposed to an ion-beam energy from 5 to 10000 eV, in particular from 50 to 5000 eV, and preferred from 200 to 1000 eV. The above-mentioned ion-beam energy is preferably intended to achieve an ion-beam current density on the surface of the porous article from 0.1 to 500 µA/cm², in particular from 1 to 250 µA/cm², preferably from 10 to 80 µA/cm². In a further embodiment, the ion-beam-assisted deposition is performed during a time period from 0.1 to 500 minutes, in particular from 1 to 200 minutes, preferably from 5 to 75 minutes. Further, the ion-beam-assisted deposition may be performed applying a deposition rate from 0.01 to 5 nm/sec, in particular from 0.1 to 2.5 nm/sec, and preferred from 0.2 to 1 nm/sec. In a particular preferred embodiment, the ion-beam-assisted deposition is carried out under the following process parameters: a vacuum pressure of at least 10⁻⁵ torr, an ion-beam energy from 200 eV to 1000 eV, an ion-beam current density from 10 to 80 µA/cm², and a deposition rate from 0.2 to 1 nm/sec.

It may be further within the scope of the present invention to coat the compressed porous article with the antimicrobial material by means of dipping, immersing, spraying, brushing and/or painting techniques. In particular, the compressed porous article may be coated by wet-chemical procedures. For example, the compressed porous article to be coated may be immersed or dipped into a liquid dispersion, suspension or solution of the antimicrobial material. Galvanic techniques are in principal also conceivable. Because of the compressing step a) preceding the coating step c), solvents, reagents, additives and the like that are typically employed in the techniques described above cannot penetrate the node and fibril microstructure that may possibly lead to undesired residues in the article and medical device, respectively.

In a further embodiment, the coated porous article is expanded subsequent to a purification step, in particular rinsing or washing step, of the article. Thus, laborious and time-consuming purifications of the node and fibril microstructure from solvents, reagents et cetera may be circumvented. Additionally, the risk of harmful residues being entrapped by the node and fibril microstructure can be lowered. Altogether, this leads to an increased biocompatibility of the porous article and the medical device, respectively.

A further disclosure relates to a medical device, preferably a vascular prosthesis, obtained or obtainable according to one of the aforedescribed methods. For further details and advantages references is made to the previous description.

In the following the present invention will be illustrated in more detail by a disclosure of preferred embodiments presented in figures, description of the figures and examples. In the embodiments, the individual features may be realized exclusively or in combination with other features. Any described embodiment is given for the sole purpose of illustration and better understanding of the invention, and is no way to be interpreted as a limitation.

The figures depict the following:
- Figure 1:: a schematically illustration of the node and fibril microstructure of expanded polytetrafluoroethylene (ePTFE),
- Figure 2:: a schematically illustration of the node and fibril microstructure of ePTFE after compressing the porous article according to the present invention
- Figure 3:: a schematically illustration of the node and fibril microstructure of ePTFE after coating the compressed porous article with an antimicrobial material according to the present invention
- Figure 4:: a schematically illustration of the coated node and fibril microstructure of ePTFE after expanding the coated porous article according to the present invention

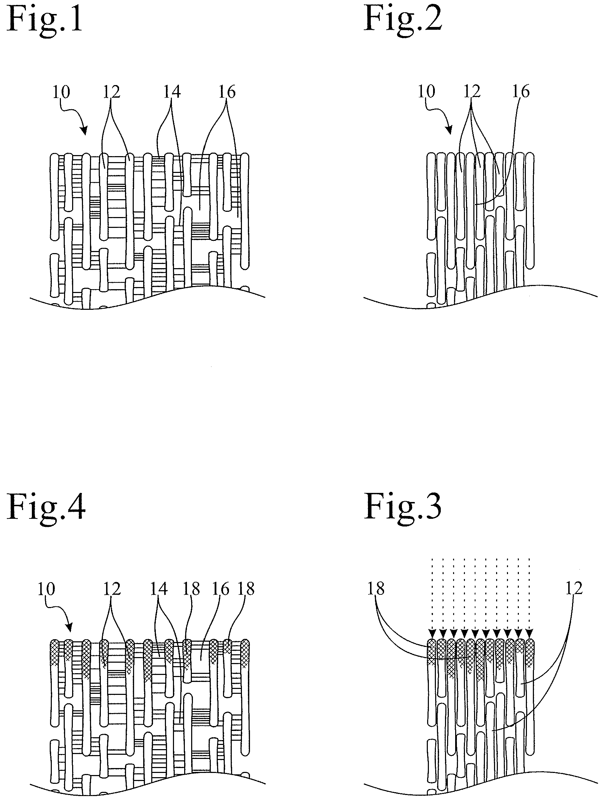

Figure 1 schematically illustrates the node and fibril microstructure 10 of expanded polytetrafluoroethylene (ePTFE) of a porous article before a compression. The microstructure 10 comprises interspaced nodes 12 that have typically an elongate shape being interconnected by fine fibrils 14. Dependent on the stretching and expanding conditions during the manufacturing process of ePTFE the form of the nodes 12 and in particular the appearance of the interconnecting fibrils 14 may vary. For example, the fibrils 14 may be bent, wavy or - as illustrated in Figure 1 - may have a straight and particularly parallel appearance.
Figure 2 schematically illustrates the node and fibril microstructure 10 of ePTFE after compressing a porous article according to the present invention. Due to the compressing step the nodes 12 are packaged densely minimizing the internodal spaces 16. Preferably, the nodes 12 are basically not interspaced any longer by the fibrils 14. Insofar, the originally open node and fibril microstructure 10 as illustrated in Figure 1 is transferred into a closed microstructure that is based on densely packaged nodes as illustrated in Figure 2.

Figure 3 schematically illustrates the node and fibril microstructure 10 of ePTFE after coating a porous article according to the present invention but before expanding the article (the coating process is depicted by dotted arrows). Basically, only nodes 12 and segments thereof, respectively at the surface of the porous article are coated with an antimicrobial material 18. The fibrils 14 at the surface are basically uncoated that is mainly due to their compression between the nodes 12 and their shielding that is associated with the compact configuration of the nodes 12. The coating 18 per se may be arranged in a certain depth, taken from the surface of the porous article.

Figure 4 schematically illustrates the node and fibril microstructure 10 of ePTFE of a porous article according to the present invention. At the surface of the article, basically only nodes 12 and segments thereof, respectively of the node and fibril microstructure 10 are coated with the antimicrobial material 18. Interconnecting fibrils 14 at the surface of the porous article are free of a coating including an antimicrobial material 18. Further, internodal spaces 16 at the surface of the porous article are also free of a coating including an antimicrobial material 18. Altogether, the porous article comprises a surface being only partially coated with the antimicrobial material 18. Nonetheless, coating the nodes 12 at the surface of the porous article is sufficient for a preferably long-term antimicrobial action of the article. Furthermore, the porous article comprises an unimpaired node and fibril microstructure 10 that is advantageous regarding tissue ingrowth into the article from its outer periphery and the prophylaxis of post-surgical complications like the formation of thrombosis and restenosis of a tubular article, preferably a vascular prosthesis.

### Example

Prostheses of ePTFE are compressed on mandrels before clamped in a rotatable clamp device so that they hang freely as a bundle of parallel tubes with spaces between them. The clamp device is introduced into a vacuum chamber suitable for carrying out an ion-beam-assisted deposition technique (IBAD technique), the ePTFE prostheses being vapordeposited with silver and at the same time bombarded with argon ions. The coating operation is conducted until a silver layer thickness of 1300 A is reached on the outside of the ePTFE prosthesis or the nodes located there. If desired, a primary coating can be effected by vapordeposition of other metals.

After the coating process the ePTFE prostheses are expanded. The ePTFE prostheses with an internal diameter of 8 mm and a wall thickness of 500 µm coated in this way each has a segment at the outer surface which is only partially coated with silver (basically only nodes or node segments at the outside surface of the prostheses are coated with silver). The silver coating has a film thickness of 130 nm resulting in a proportion of silver relative to the total weight in the range from 0,3 to 0,5 % by weight which is equivalent to a proportion of 0,01 to 0,04 % in relation to the wall thickness of the porous ePTFE prostheses. The coating is applied to the ePTFE prostheses in a depth of 40 to 80 µm.

## Claims

1. A medical device comprising a porous article of expanded polytetrafluoroethylene (ePTFE) having a microstructure of nodes interconnected by fibrils and a coating including an antimicrobial material at the surface of the porous article, **characterized in that** the microstructure at the surface of the porous article is only partially coated with the antimicrobial material, wherein only nodes or segments thereof at the surface of the porous article are coated with the antimicrobial material and fibrils at the surface of the porous article are at most partially coated with the antimicrobial material.

2. The medical device according to claim 1, **characterized in that** the nodes or segments thereof are completely coated with the antimicrobial material.

3. The medical device according to claim 1 or 2, **characterized in that** the fibrils are completely free of a coating including an antimicrobial material.

4. The medical device according to one of the preceding claims, **characterized in that** the coating including an antimicrobial material leaves internodal spaces open at the surface of the porous article.

5. The medical device according to one of the preceding claims, **characterized in that** the coating including an antimicrobial material has a proportion from 0.01 to 5.0 % by weight, in particular from 0.1 to 3.0 % by weight, and preferred from 0.3 to 1.5 % by weight, relating to the total weight of the porous article.

6. The medical device according to one of the preceding claims, **characterized in that** the coating has a layer thickness from 10 to 400 nm, in particular from 40 to 250 nm, and preferred from 80 to 160 nm.

7. The medical device according to one of the preceding claims, **characterized in that** the antimicrobial material is selected from the group consisting of an antimicrobial metal, antimicrobial alloy, antimicrobial compound, in particular salt, thereof or combinations thereof.

8. The medical device according to one of the preceding claims, **characterized in that** the antimicrobial material is selected from the group consisting of zirconium, copper, zinc, silver, gold, palladium, platinum, iridium, aluminium, nickel, tungsten, molybdenum, tantalum, titanium, iodine, alloys thereof, compounds thereof, salts thereof and combinations thereof.

9. The medical device according to one of the preceding claims, **characterized in that** the porous article is a tubular article.

10. The medical device according to claim 9, **characterized in that** only nodes at the outside surface of the tubular article are coated with the antimicrobial material.

11. The medical device according to one of the preceding claims, **characterized in that** the device is a tubular prosthesis, preferably an arterial prosthesis or a vein prosthesis, more preferably an arterial prosthesis.

12. A method for the manufacture of a medical device according to one of the preceding claims comprising the steps of
a) compressing a porous article, preferably a tubular porous article, of ePTFE having a microstructure of nodes interconnected by fibrils,
b) coating the compressed porous article, preferably tubular porous article, with an antimicrobial material,
c) expanding the coated porous article, preferably tubular porous article.

13. The method according to claim 12, **characterized in that** the porous article is compressed on a mandrel, in particular a rotatable mandrel.

14. The method according to claim 12 or 13, **characterized in that** the compressed porous article is coated by means of a dry coating process, in particular by means of a physical vapour deposition, preferably by means of an ion-beam-assisted deposition.

## Patentansprüche

1. Medizinische Vorrichtung, umfassend einen Porenkörper aus expandiertem Polytetrafluorethylen (ePTFE ), der eine Mikrostruktur von durch Fibrillen miteinander verbundenen Knoten aufweist, und eine Beschichtung mit einem antimikrobiellen Material auf der Oberfläche des Porenkörpers,
**dadurch gekennzeichnet, dass**
die Mikrostruktur an der Oberfläche des Porenkörpers nur teilweise mit dem antimikrobiellen Material beschichtet ist, wobei nur Knoten oder Segmente davon an der Oberfläche des Porenkörpers mit dem antimikrobiellen Material beschichtet sind und Fibrillen an der Oberfläche des Porenkörpers höchstens teilweise mit dem antimikrobiellen Material beschichtet sind.

2. Medizinische Vorrichtung nach Anspruch 1, **dadurch gekennzeichnet, dass** die Knoten oder Segmente davon vollständig mit dem antimikrobiellen Material beschichtet sind.

3. Medizinische Vorrichtung nach Anspruch 1 oder 2, **dadurch gekennzeichnet, dass** die Fibrillen vollständig frei von einer Beschichtung mit einem antimikrobiellen Material sind.

4. Medizinische Vorrichtung nach einem der vorhergehenden Ansprüche, **dadurch gekennzeichnet, dass** die Beschichtung mit einem antimikrobiellen Material an der Oberfläche des Porenkörpers zwischen den Knoten Lücken offen lässt.

5. Medizinische Vorrichtung nach einem der vorhergehenden Ansprüche, **dadurch gekennzeichnet, dass** die Beschichtung mit einem antimikrobiellen Material in einem Anteil von 0,01 bis 5,0 Gewichts-%, insbesondere von 0,1 bis 3,0 Gewichts-%, und bevorzugt von 0,3 bis 1,5 Gewichts-%, bezogen auf das Gesamtgewicht des Porenkörpers vorliegt.

6. Medizinische Vorrichtung nach einem der vorhergehenden Ansprüche, **dadurch gekennzeichnet, dass** die Beschichtung eine Schichtdicke von 10 bis 400 nm, insbesondere von 40 bis 250 nm, und bevorzugt von 80 bis 160 nm aufweist.

7. Medizinische Vorrichtung nach einem der vorhergehenden Ansprüche, **dadurch gekennzeichnet, dass** das antimikrobielle Material ausgewählt ist aus der Gruppe bestehend aus einem antimikrobiellen Metall, einer antimikrobiellen Legierung, einer antimikrobiellen Verbindung, insbesondere einem Salz davon oder Kombinationen davon.

8. Medizinische Vorrichtung nach einem der vorhergehenden Ansprüche, **dadurch gekennzeichnet, dass** das antimikrobielle Material ausgewählt ist aus der Gruppe bestehend aus Zirconium, Kupfer, Zink, Silber, Gold, Palladium, Platin, Iridium, Aluminium, Nickel, Wolfram, Molybdän, Tantal, Titan, Iod, Legierungen davon, Verbindungen davon, Salzen davon oder Kombinationen davon.

9. Medizinische Vorrichtung nach einem der vorhergehenden Ansprüche, **dadurch gekennzeichnet, dass** der Porenkörper als rohrförmiger Körper ausgebildet ist.

10. Medizinische Vorrichtung nach Anspruch 9, **dadurch gekennzeichnet, dass** nur Knoten an der Außenseite des rohrförmigen Körpers mit dem antimikrobiellen Material beschichtet sind.

11. Medizinische Vorrichtung nach einem der vorhergehenden Ansprüche, **dadurch gekennzeichnet, dass** die Vorrichtung als rohrförmige Prothese, bevorzugt als Arterienprothese oder Venenprothese, besonders bevorzugt als Arterienprothese ausgebildet ist.

12. Verfahren zur Herstellung einer medizinischen Vorrichtung nach einem der vorhergehenden Ansprüche, umfassend die Schritte
a) Komprimieren eines Porenkörpers, bevorzugt eines rohrförmigen Porenkörpers, aus ePTFE, der eine Mikrostruktur von durch Fibrillen miteinander verbundenen Knoten aufweist,
b) Beschichten des komprimierten Porenkörpers, bevorzugt des rohrförmigen Porenkörpers, mit einem antimikrobiellen Material,
c) Expandieren des beschichteten Porenkörpers, bevorzugt des rohrförmigen Porenkörpers.

13. Verfahren nach Anspruch 12, **dadurch gekennzeichnet, dass** der Porenkörper auf einem Dorn, insbesondere einem drehbaren Dorn, komprimiert wird.

14. Verfahren nach Anspruch 12 oder 13, **dadurch gekennzeichnet, dass** der komprimierte Porenkörper mittels eines Trockenbeschichtungsprozesses, insbesondere mittels einer physikalischen Gasphasenabscheidung, bevorzugt mittels einer ionenstrahlgestützten Abscheidung, beschichtet wird.

## Revendications

1. Dispositif médical comprenant un article poreux en polytétrafluoroéthylène expansé (ePTFE) comportant une microstructure de noeuds connectés les uns aux autres par des fibrilles et un revêtement contenant un matériau antimicrobien sur la surface de l'article poreux, **caractérisé en ce que** la microstructure sur la surface de l'article poreux est seulement en partie revêtue du matériau antimicrobien, dans lequel seulement les noeuds ou des segments de ceux-ci sur la surface de l'article poreux sont revêtus du matériau antimicrobien et les fibrilles sur la surface de l'article poreux sont tout au plus en partie revêtues du matériau antimicrobien.

2. Dispositif médical selon la revendication 1, **caractérisé en ce que** les noeuds ou des segments de ceux-ci sont entièrement revêtus du matériau antimicrobien.

3. Dispositif médical selon la revendication 1 ou 2, **caractérisé en ce que** les fibrilles sont entièrement exemptes de revêtement contenant un matériau antimicrobien.

4. Dispositif médical selon l'une des revendications précédentes, **caractérisé en ce que** le revêtement contenant un matériau antimicrobien laisse les espaces internodaux ouverts sur la surface de l'article poreux.

5. Dispositif médical selon l'une des revendications précédentes, **caractérisé en ce que** le revêtement contenant un matériau antimicrobien représente une proportion de 0,01 à 5,0 % en poids, en particulier de 0,1 à 3,0 % en poids, et préférablement de 0,3 à 1,5 % en poids, relativement au poids total de l'article poreux.

6. Dispositif médical selon l'une des revendications précédentes, **caractérisé en ce que** le revêtement a une épaisseur de couche de 10 à 400 nm, en particulier de 40 à 250 nm, et préférablement de 80 à 160 nm.

7. Dispositif médical selon l'une des revendications précédentes, **caractérisé en ce que** le matériau antimicrobien est sélectionné dans le groupe constitué d'un métal antimicrobien, d'un alliage antimicrobien, d'un composé antimicrobien, en particulier d'un sel de ceux-ci ou de combinaisons de ceux-ci.

8. Dispositif médical selon l'une des revendications précédentes, **caractérisé en ce que** le matériau antimicrobien est sélectionné dans le groupe constitué du zirconium, du cuivre, du zinc, de l'argent, de l'or, du palladium, du platine, de l'iridium, de l'aluminium, du nickel, du tungstène, du molybdène, du tantale, du titane, de l'iode, d'alliages de ceux-ci, de composés de ceux-ci, de sels de ceux-ci et de combinaisons de ceux-ci.

9. Dispositif médical selon l'une des revendications précédentes, **caractérisé en ce que** l'article poreux est un article tubulaire.

10. Dispositif médical selon la revendication 9, **caractérisé en ce que** seulement des noeuds situés sur la surface extérieure de l'article tubulaire sont revêtus du matériau antimicrobien.

11. Dispositif médical selon l'une des revendications précédentes, **caractérisé en ce que** le dispositif est une prothèse tubulaire, préférablement une prothèse artérielle ou une prothèse veineuse, plus préférablement une prothèse artérielle.

12. Procédé de fabrication d'un dispositif médical selon l'une des revendications précédentes, comprenant les étapes qui consistent à :
a) comprimer un article poreux, préférablement un article poreux tubulaire, en ePTFE comportant une microstructure de noeuds connectés les uns aux autres par des fibrilles,
b) revêtir l'article poreux comprimé, préférablement l'article poreux tubulaire, d'un matériau antimicrobien,
c) expanser l'article poreux, préférablement l'article poreux tubulaire, qui a été revêtu.

13. Procédé selon la revendication 12, **caractérisé en ce que** l'article poreux est comprimé sur un mandrin, en particulier un mandrin rotatif.

14. Procédé selon la revendication 12 ou 13, **caractérisé en ce que** l'article poreux comprimé est revêtu par un procédé de revêtement à sec, en particulier par un procédé de dépôt physique en phase vapeur, préférablement par un procédé de dépôt assisté par ions.
